(19) Europäisches Patentamt
European Patent Office
Office européen des brevets

(11) **EP 1 721 637 A1**

(12) **EUROPEAN PATENT APPLICATION**

(43) Date of publication:
**15.11.2006 Bulletin 2006/46**

(51) Int Cl.:
*A61Q 19/00* (2006.01)    *A61K 8/02* (2006.01)
*A61K 8/37* (2006.01)    *A61K 8/81* (2006.01)

(21) Application number: **06008975.2**

(22) Date of filing: **28.04.2006**

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(30) Priority: **09.05.2005 JP 2005135722**
**15.08.2005 JP 2005235286**
**02.09.2005 JP 2005255270**

(71) Applicant: **NITTO DENKO CORPORATION Osaka (JP)**

(72) Inventors:
• **Konno, Eriko**
**Ibaraki-shi**
**Osaka (JP)**

• **Kasahara, Tsuyoshi**
**Ibaraki-shi**
**Osaka (JP)**
• **Hatanaka, Hiroshi**
**Ibaraki-shi**
**Osaka (JP)**
• **Funahashi, Miki**
**Ibaraki-shi**
**Osaka (JP)**

(74) Representative: **Grünecker, Kinkeldey, Stockmair & Schwanhäusser Anwaltssozietät Maximilianstrasse 58 80538 München (DE)**

(54) **Cosmetic sheet for removing keratotic plugs**

(57) A cosmetic sheet which comprises a support and, provided on one surface of the support, a skin-attaching layer comprising a wet pressure-sensitive adhesive composition containing a water-soluble polymer and a polyoxyethylene sorbitan fatty acid ester, wherein the polyoxyethylene sorbitan fatty acid ester has oozed out on the surface of the skin-attaching layer. The cosmetic sheet does not impart pain at the skin and can sufficiently remove keratotic plugs.

## FIG. 1

CHANGE IN CONTACT ANGLE AGAINST WATER WITH TIME

—•— REFERENCE EXAMPLE
—♦— EXAMPLE 1
—▲— COMPARATIVE EXAMPLE 1
—■— COMPARATIVE EXAMPLE 2

CONTACT ANGLE (C.A.) [°]

CONTACT TIME [μm]

EP 1 721 637 A1

## Description

FIELD OF THE INVENTION

The present invention relates to a cosmetic sheet. Specifically, it relates to a cosmetic sheet to be used for removing keratotic plugs and the like.

BACKGROUND OF THE INVENTION

Recently, there is a known cosmetic sheet suitable for removing keratotic plugs from the skin, wherein a skin-attaching layer using a water-soluble polymer is formed on one surface of a support layer composed of a non-woven fabric or the like. The cosmetic sheet is usually attached after a site to be attached (e.g., a nose part) is wetted with water. The cosmetic sheet attached is allowed to be left under such conditions for about 10 minutes and, after voporization of moisture, it is slowly peeled and removed. During such a series of operations, the skin-attaching layer dissolved in water or wetted with water is brought into contact with keratotic plugs present in the pores and then dried, so that the layer is integrated with the keratotic plugs and hence the keratotic plugs can be removed together by peeling off the skin-attaching layer.

Since the cosmetic sheet is peeled off from the skin in a state that the sheet is already dried and bonded or adhered to the skin, considerable pain is imparted to the skin at the time of peeling. JP-A-5-221843 discloses a sheet-like pack using a polymer compound having a salt-forming group and an oil agent as one component of the pack as a means for removing keratotic plugs without imparting pain to the skin. However, there is a possibility of lowering keratotic plug-removing ability since it uses an oil agent in combination. On the other hand, JP-A-11-199435 discloses a pack cosmetic capable of reducing pain at the time of peeling off from the skin without lowering keratotic plug~removing ability of the pack. The pack cosmetic contains one or two or more components selected from the group consisting of an analgesic, an antipruritic, a nonionic surfactant having a polyoxyalkylene chain, a salicylic acid ester of an alkylene glycol or a polyalkylene glycol, and a condensate of a higher fatty acid with an amino alcohol.

However, the pack cosmetic still does not have sufficient keratotic plug-removing ability and cannot meet high needs of users. Namely, users have not satisfied with the keratotic plug-removing ability of conventional cosmetic sheets and thus it is desired to develop a cosmetic sheet having more excellent removing ability.

The present invention was accomplished taking the above problems into consideration and an object of the present invention is to provide a cosmetic sheet which does not impart pain to the skin and can sufficiently remove keratotic plugs.

Patent Document 1: JP-A-5-221843
Patent Document 2: JP-A-11-199435

## SUMMARY OF THE INVENTION

The present invention is to solve the above problems and an object of the present invention is to provide a cosmetic sheet which reduces pain at the time of peeling off from the skin and has more excellent keratotic plug-removing ability from the skin than conventional ones.

The present inventors have studied and developed a technology for improving the keratotic plug-removing ability of cosmetic sheets. In the progress of the studies, they have found that keratotic plugs can be removed without imparting pain to the skin and an excellent keratotic plug-removing ability can be exhibited by incorporating a polyoxyethylene sorbitan fatty acid ester into a skin-attaching layer and allowing the polyoxyethylene sorbitan fatty acid ester to ooze out on the skin-attaching surface. Based on this finding, they have accomplished the present invention.

Namely, the present invention includes the following embodiments.

1. A cosmetic sheet which comprises a support and, provided on one surface of the support, a skin-attaching layer comprising a wet pressure-sensitive adhesive composition containing a water-soluble polymer and a polyoxyethylene sorbitan fatty acid ester, wherein the polyoxyethylene sorbitan fatty acid ester has oozed out on the surface of the skin-attaching layer.

2. The cosmetic sheet according to the above 1, wherein the polyoxyethylene sorbitan fatty acid ester which has oozed out on the surface of the skin-attaching layer exists as a layer.

3. The cosmetic sheet according to the above 1, wherein said wet pressure-sennitive adhesive composition further comprises a filler.

4. The cosmetic sheet according to the above 3, wherein said filler is at least one selected from the group consisting of silica, alumina, titanium dioxide, titanium black, carbon black, black iron oxide, medicated carbon, aluminum hydroxide, magnesium hydroxide, calcium carbonate, and talc.

5. The cosmetic sheet according to the above 3, wherein said inorganic filler has an average particle size of 0.01 to 50 $\mu$m.

6. The cosmetic sheet according to the above 1 or 2, wherein the fatty acid forming said polyoxyethylone sorbitan fatty acid ester is one selected from the group consisting of lauric acid, palmitic acid, stearic acid, and oleic acid.

7. The cosmetic sheet according to the above 1 or 2, wherein HLB of said polyoxyethylene sorbitan fatty acid ester is 12 or more.

8. The cosmetic sheet according to the above 1, wherein said water-soluble polymer is one selected from the group consisting of polyvinylpyrrolidone and vinylpyrrolidone-vinyl acetate copolymers.

9. The cosmetic sheet according to the above 1, wherein said water-soluble polymer comprises a water-soluble polymer having a weight-average molecular weight of less than 700,000 and a water-soluble polymer having a weight-average molecular weight of 700,000 or more.

10. The cosmetic sheet according to the above 1, wherein said wet pressure-sensitive adhesive composition further comprises a moisturizing component.

11. The cosmetic sheet according to the above 10, wherein said moisturizing component is one selected from the group consisting of glycerol and polyhydric alcohols.

12. The cosmetic sheet according to the above 1, wherein said support is a moisture-permeable support.

13. The cosmetic sheet according to the above 1, which further comprises a sheet as a separator on the surface of said skin-attaching layer.

14. The cosmetic sheet according to the above 13, wherein the sheet as a separator is a sheet which has not been subjected to release treatment.

15. The cosmetic sheet according to the above 13, wherein said sheet is one selected from the group consisting of plastic films and paper.

16. The cosmetic sheet according to the above 14, wherein said plastic film comprises one selected from the group consisting of polyethylene, polypropylene, and polyesters.

The present invention provides a cosmetic sheet which can reduce pain at the time of peeling off from the skin and has more excellent keratotic plug-removing ability from the skin than conventional ones.

**BRIEF DESCRIPTION OF THE DRAWINGS**

By way of example and to make the description more clear, reference is made to the accompanying drawings in which:

Fig. 1 is a graph showing change in contact angle against water with time in the skin-attaching layer of the cosmetic sheet.

Fig. 2 is a graph showing change in contact angle against oleic acid with time in the skin-attaching layer of the cosmetic sheet.

Fig. 3 is a graph showing results of comparing keratotic plug-removing ability of the cosmetic sheets of Comparative Examples 1 and 2 with that of the cosmetic sheet of Example 1.

Fig. 4 is a graph showing results of comparing keratotic plug-removing ability of the cosmetic sheets of Example 1 and Comparative Example 2 with that of the cosmetic sheet of Comparative Example 1.

Fig. 5 is a graph showing results of comparing keratotic plug-removing ability of the cosmetic sheets of Example 1 and Comparative Example 1 with that of the cosmetic sheet of Comparative Example 2.

Fig. 6 is a drawing schematically showing the layer constitution of the cosmetic sheet according to the one embodiment of the present invention. In Fig. 6, the numbers respectively have the following meanings.

1: Support
2: Skin-attaching layer
3: Polyoxyethylene sorbitan fatty acid ester which has oozed out.

Fig. 7 is a drawing showing results of evaluation of the keratotic plug-removing ability with respect to the cosmetic sheets of Example 2 and Comparative Example 1.

Fig. 8 is a graph showing results of measurements of the separator peeling force with respect to the cosmetic sheets of Example 3, Comparative Example 3, and Comparative Example 4.

DETAILED DESCRIPTION OF THE INVENTION

The cosmetic sheet of the present invention has a skin-attaching layer on one surface of a support, wherein the skin-attaching layer is formed with using a wet pressure-sensitive adhesive composition containing a water-soluble polymer and a polyoxyethylene sorbitan fatty acid ester.

It is necessary for the cosmetic sheet of the present invention that the polyoxyethylene sorbitan fatty acid ester has oozed out on the surface of the skin-attaching layer. Furthermore, the polyoxyethylene sorbitan fatty acid ester preferably forms a layer.

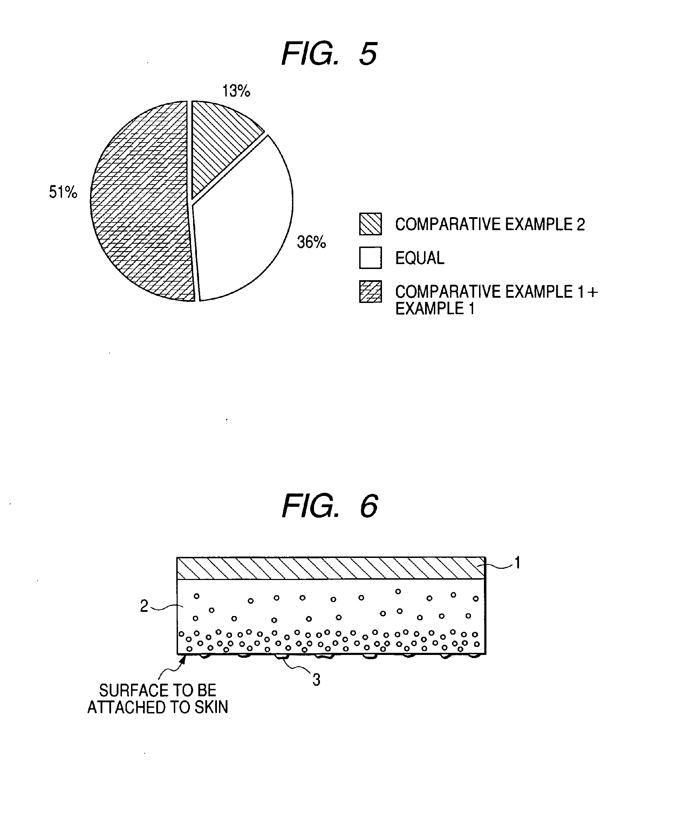

For example, as shown in Fig. 6, the cosmetic sheet of the present invention has a skin-attaching layer 2 on one surface of a moisture-permeable support 1 and a polyoxyethylene sorbitan fatty acid ester is unevenly distributed to the opposite side of the support of the skin-attaching layer 2 (skin-attaching side). Furthermore, as shown in the figure, the polyoxyethylene sorbitan fatty acid ester has oozed out at the surface part opposite to the support (surface at the skin-attaching side) and a part thereof may be present as a oozed out part 3 of the polyoxyethylene sorbitan fatty acid ester on the surface. The oozed out part (bled-out part) preferably forms a layer. In Fig. 6, a separator is not shown but a separator may be provided in some cases so that it covers the surface of the skin-attaching layer 2.

In the cosmetic sheet of the present invention, the polyoxyethylene sorbitan fatty acid ester has oozed out on the surface of the skin-attaching layer. The layer of the polyoxyethylene sorbitan fatty acid ester can be formed on the skin-attaching layer by suitably controlling the kind and blending amount of the water-soluble polymer, the blending amount of the polyoxyethylene sorbitan fatty acid ester, and/or the kind and blending amount of the inorganic filler.

One means for making the polyoxyethylene sorbitan fatty acid ester to ooze out is to incorporate a filler into the wet pressure-sensitive adhesive composition to be used for forming the skin-attaching layer. However, as the filler to be used herein, use of a material having a good affinity to the polyoxyethylene sorbitan fatty acid ester may cause difficulty in oozing out of the polyoxyethylene sorbitan fatty acid ester. Examples of the filler having a good affinity to the polyoxyethylene sorbitan fatty acid ester include fillers which exhibits basicity and is hydrophilic, for example, triiron tetroxide, calcium carbonate, aluminum hydroxide, magnesium hydroxide, and the like. Therefore, it is preferable for the present invention not to incorporate these fillers into the wet pressure-sensitive adhesive composition. When these fillers are used, it is preferable to use it in a relatively small amount. On the other hand, fillers exhibiting acidity are most preferably used as the filler.

By incorporating a filler into the wet pressure-sensitive adhesive composition, mechanical strength of the formed skin-attaching layer after wetting and drying can be also enhanced. Moreover, by incorporating a filler, the time required for drying after attachment can be shortened. Therefore, when a filler is incorporated, a long period of time as is the case of conventional products is not required even when the attaching layer is thick. The reason why the time required for drying is shortened is assumed to be that the interface between the filler and the water-soluble polymer and a liquid plasticizer functions as a pathway for moisture dissipation.

The filler is usually used preferably within the range of 10 to 200 parts by weight, more preferably within the range of 25 to 100 parts by weight relative to 100 parts by weight of the water-soluble polymer. When the amount of the filler to be used is within the range of 10 to 200 parts by weight, there may not occur a case that the skin-attaching layer formed is poor in flexibility and hence is difficult to fit well to a site to be attached. In addition, the time required for drying is not prolonged and an improved effect on mechanical strength of the skin-attaching layer after attachment and drying is obtained.

As the filler, an inorganic filler and an organic filler may be mentioned and it is preferable to use an inorganic filler.

Examples of the inorganic filler constituting the wet pressure-sensitive adhesive composition according to the present invention include silica, alumina, titanium dioxide, titanium black, carbon black, black iron oxide, medicated carbon, aluminum hydroxide, magnesium hydroxide, calcium caxbonate, and talc, and the like. These inorganic fillers may be used solely or two or more thereof may be used in combination. Usually, the filler to be used in the present invention is preferably in a powder form. The shape Is not particularly limited but in preferably spherical in view of obtaining a homogeneous dispersing ability. Moreover, the average particle size thereof is preferably from 0.01 to 50 μm, more preferably from 0.1 to 10 μm.

in order to make the polyoxyethylene sorbitan fatty acid ester to ooze out on the surface part of the skin-attaching layer, the inorganic filler constituting the wet pressure-sensitive adhesive composition according to the present invention is preferably one selected from the group consisting of silica, alumina, titanium oxide, titanium black, and medicated carbon, among the above-described fillers. Through oozing of the polyoxyethylene sorbitan fatty acid ester on the above surface part, the polyoxyethylene sorbitan fatty acid ester which has oozed on the surface part is apt to come into contact with keratotic plugs present in the pores when the cosmetic sheet is attached to the skin. Since the polyoxyethylene sorbitan fatty acid ester is highly compatible to sebaceous components such as cholesterol and waste products attached to the pores, an excellent keratotic plug-removing ability can be exhibited.

In the present invention, one or more fillers may be selected from the above-described inorganic fillers, and medicated carbon and a part thereof can be blended as a pigment. The amount of the inorganic filler to be blended as a pigment is preferably within the range of 1 to 20 parts by weight, more preferably within the range of 3 to 10 parts by weight relative to 100 parts by weight of the water-soluble polymer. When the amount of the inorganic filler used as a pigment is within the range of 1 to 20 parts by weight, a skin-attaching layer showing good white or black color can be formed.

The polyoxyethylene sorbitan fatty acid ester to be used for forming the skin-attaching layer is preferably blended in the range of 3 parts by weight to 40 parts by weight, more preferably in the range of 5 parts by weight to 20 parts by weight, particularly preferably in the amount of about 10 parts by weight relative to 100 parts by weight of the water-soluble polymer. When the amount of the polyoxyethylene sorbitan fatty acid ester to be blended is 3 parts by weight or more, it is possible to allow it to ooze out on the surface of the skin-attaching layer and the keratotic plug-removing ability can be sufficiently exhibited. Moreover, when the amount of the polyoxyethylene sorbitan fatty acid ester to be blended is 40 parts by weight or less, the polyoxyethylene sorbitan fatty acid ester is not reserved in a large amount at the interface between the skin-attaching layer and the skin and the removal of the keratotic plugs is not inhibited.

The polyoxyethylene sorbitan fatty acid ester is a nonionic surfactant composed of a higher fatty acid ester of sorbit and is an oil-in-water type (o/w type), which emulsifies an oil in water. Moreover, as a numeric value showing a correlation between a hydrophilic group and a lipophilic group in a surfactant molecule, there is HLB (Hydrophilic Lipophilic Balance). The HLB is preferably 12 or more and the upper limit is preferably 20 or less. It is more preferably from 14 to 17, particularly preferably from 15 to 17. The polyoxyethylene sorbitan fatty acid ester to be used in the present invention is highly compatible to sebaceous components such as cholesterol and waste products attached to the scalp and pores of human bodies.

Examples of the preferable fatty acid in the polyoxyethylone sorbitan fatty acid ester to be used in the present invention include lauric acid, palmitic acid, stearic acid, and oleic acid. Moreover, for the polyoxyethylene sorbitan fatty acid ester, the addition mol number of ethylene oxide (EO) is preferably from 4 mol to 20 mol, for example.

The water-soluble polymer to be used in the present invention exhibits pressure-sensitive adhesiveness by the presence of water or a hydrophilic medium. Examples of the preferable water-soluble polymer suitable for keratotic plug-removal is one selected from the group consisting of polyvinylpyrrolidone and vinylpyrrolidone-vinyl acetate copolymers. Moreover, when the molecular weight of the water-soluble polymer is too large, the skin-attaching layer formed using the wet pressure-sensitive adhesive composition may not have a sufficient adhesive force in some cases even when water or the hydrophilic medium is present. When the molecular weight is too small, mechanical strength of the skin-attaching layer may decrease in some cases and, after attachment, a part of the skin-attaching layer may be left as a residue at the time when a dried sheet-like attaching material is removed by peeling from the site attached. Therefore, the water-soluble polymer preferably has a weight-average molecular weight of 5,000 to 5,000,000 and more preferably from 20,000 to 1,200,000.

From the viewpoint of shortening drying time for vaporization of water or the like used for the purpose of exhibition of adhesiveness and of enabling further reduction of pain at peeling, it is more preferable to use a low-molecular-weight water-soluble polymer, i.e., a water-soluble polymer having a weight-average molecular weight of less than 700,000, preferably from 5,000 to 300,000 and a high-molecular-weight water-soluble polymer, i.e., a water-soluble polymer having a weight-average molecular weight of 700,000 or more, preferably from 1,000,000 to 5,000,000 in combination. In the case that a low-molecular-weight water-soluble polymer and a high-molecular-weight water-soluble polymer are used in combination, it is preferable to suitably determine the blending ratio thereof according to the molecular weight and intended use of the water-soluble polymers used. For example, the ratio of the high-molecular-weight water-soluble polymer to the low-molecular-weight water-soluble polymer is preferably from about 5:95 to 95:5, more preferably from 20:80 to 70:30. In view of alleviating pain imparted to the skin at the time of peeling, it is preferable to use the low-molecular-weight water-soluble polymer in larger ratio. In the present invention, it is preferable to use polyvinylpyrrolidone having a weight-average molecular weight of 1,200,000 and polyvinylpyrrolidone having a weight-average molecular weight of 450,000 in combination. When they are used in combination, polyvinylpyrrolidone having a weight-average molecular weight of 1,200,000 and polyvinylpyrrolidone having a weight-average molecular weight of 450,000 are used in combination in a weight ratio of preferably within the range of 30:70 to 60:40, more preferably within the range of 40:60 to 50:50.

A moisturizing component may be blended in the wet pressure-sensitive adhesive composition according to the present invention. Examples of the moisturizing component to be used in the present invention include a material which is compatible to the water-soluble polymer and shows a plasticizing effect through dissolution. Illustrative examples thereof include ethylene glycol, diethylene glycol, triethylene glycol, hexamethylene glycol, and the other polyethylene glycols; propylene, glycol, dipropylene glycol, and the other polypropylene glycols; glycerol, diglycerol, and the other polyglycerols; butylene glycols such as 1,3-butylene glycol and 1,4-butylene glycol; sugar alcohols such as sorbitol and mannitol; glycerides such as lanoline, lecithin, and olive oil; and the like. These compounds may be used solely or two or more thereof may be used in combination.

When the amount of the moisturizing component to be used is too large, the mechanical strength of the formed skin-attaching layer after wetting and drying is apt to be poor. When the amount is too small, the layer may become poor in flexibility and hence tends to become difficult to fit to a site to be attached. Therefore, the moisturizing component is used preferably in the range of 1 to 75 parts by weight, more preferably in the range of 5 to 50 parts by weight.

If necessary, the wet pressure-sensitive adhesive composition according to the present invention may contain a cosmetic, a fragrant material, a preservative, a colorant, an alcohol, a medical agent, a UV absorbent, or the other medical agent or additive to be usually used in cosmetic sheets in the range where the advantage of the present invention is not inhibited.

The wet pressure-sensitive adhesive composition which contains the water-soluble polymer and the polyoxyethylene sorbitan fatty acid ester and, if necessary, a filler, a moisturizing component, and the like is converted into a coating solution together with water or a hydrophilic medium such as ethyl alcohol or methyl alcohol and then the solution is applied on a release sheet (may have been subjected to release treatment) or a support and dried to form a sheet-like skin-attaching layer.

In the present invention, the thickness of the skin-attaching layer is preferably from 10 $\mu$m to 500 $\mu$m, more preferably from 50 $\mu$m to 250 $\mu$m.

The cosmetic sheet of the present invention has a skin-attaching layer on one surface of the support. As the support, it is preferable to use a moisture-permeable support which is moisture permeable from the viewpoint of drying rate of the skin-attaching layer. As its structure, aggregates of fibers, such as woven fabrics, non-woven fabrics, knit fabrics, and paper, and films such as porous films and gas-permeable films may be mentioned as examples. Of these, those having an appropriate elasticity which are apt to be fit to curved surface of the site to be attached are particularly preferred. Moreover, with regard to the material, synthetic or natural organic polymers such as nylons, polyesters, polyethylene, polypropylene, polyurethanes, and celluloses may be mentioned as examples.

Furthermore, the thickness of the support is preferably from 10 $\mu$m to 200 $\mu$m.

The cosmetic sheet of the present invention may have a separator on the skin-attaching layer. As the separator to be used in the present invention, a plastic sheet or film, a paper, or the like may be used.

Examples of the material forming such a plastic sheet include polyethylene, polypropylene, polyesters, and the like and polyethylene is preferably used. In the present invention, the plastic sheet may be formed using two or more materials in combination. Moreover, the plastic sheet may be a laminate composed of two or more layers or may be a laminate made of a paper and a plastic sheet.

As the separator, it is possible to use a sheet whose surface has been subjected to release treatment but a sheet whose surface has not been subjected to release treatment is preferable. Namely, the separator to be used in the present invention is preferably a sheet which is not subjected to surface-treatment with a polymer or the like (e.g., silicone). Examples of the sheet include plastic films, papers, and the like whose surface has not been subjected to release treatment.

The cosmetic sheet of the present invention can be formed, for example, by applying a coating solution obtained by dispersing a wet pressure-sensitive adhesive composition in water or the like on one surface of a sheet (separator), overlaying a support thereon, and drying the whole. The cosmetic sheet has a constitution of support/skin-attaching layer/separator and is cut into any shapes. The separator can protect the skin-attaching layer from the viewpoint of hygiene and adhesive force until peeled at use. Moreover, the presence of the separator affords an advantage that the cosmetic sheet can be stored in a sheet-stacked state.

The cosmetic sheet of the present invention may be wrapped with a moisture-impermeable support in order to prevent evaporation of moisture in the cosmetic sheet. For example, it may be air-tightly stored by means of a bag made of a moisture-impermeable support or the like. As the moisture-impermeable support, a foil of a metal such as aluminum is preferably used, for example. The support may be a laminate with a plastic film and, for example, a laminate of aluminum with a polyester film or the like is preferably used.

The cosmetic sheet of the present invention may be stored in a state where the sheet is impregnated with water or a hydrophilic medium beforehand. In this case, the cosmetic sheet may be attached as it is without wetting a site to be attached with water or the like.

When the cosmetic sheet of the present invention is attached to the skin, the polyoxyethylene sorbitan fatty acid ester is reserved at the interface between the skin-attaching layer and the skin, so that the skin surface exhibiting oily nature can be rapidly wetted therewith and hence the keratotic plug-removing ability can be enhanced. Moreover, when a filler is incorporated, a cosmetic sheet also excellent in mechanical strength can be realized.

Since the cosmetic sheet of the present invention contains the polyoxyethylene sorbitan fatty acid ester, which is a nonionic surfactant, in the skin-attaching layer, pain at the time of peeling from the skin can be reduced and also keratotic plugs can be sufficiently removed.

The cosmetic sheet of the present invention is useful as a keratotic plug-removing sheet, an acne sheet, and the like as represented by a peel-off type pack or the like. In particular, it can be closely attached to intricately curved skin such as the nose part and hence can be effectively utilized as a keratotic plug-removing sheet at the nose part.

The following will explain the present invention in detail with reference to Examples but the invention should not be construed as being limited thereto and various variations are possible within the scope not deviating from the technical concept of the invention.

(Example 1)

Using 58% by weight of polyvinylpyrrolidone having a weight-average molecular weight of 1,200,000 as a water-soluble polymer, 6% by weight of a sorbitan oleic acid monoester (ethylene oxide 20 mol adduct, HLB 15.0) as a polyoxyethylene sorbitan fatty acid ester, 34% by weight of silicic anhydride, and 2% by weight of titanium oxide, these compounds were stirred and mixed with an appropriate amount of purified water to prepare a coating solution. The coating solution was applied on the release-treated surface of a polyethylene film (thickness 50 $\mu$m), whose one surface had been subjected to silicone treatment, so as to achieve an applied amount of 110 g/m$^2$ and a uniform thickness, whereby a skin-attaching layer was formed. A spun lace non-woven fabric (basis weight: 40 g/m$^2$) made of polyester fibers was overlaid on the skin-attaching layer and the whole was dried at 105°C for 3 minutes to prepare a cosmetic sheet having a three-layer structure. The moisture content of the resulting cosmetic sheet was found to be from 15 to 20% by weight. The moisture content was calculated based on the weight change after moisture control for about 15 minutes under the environment of a temperature of 30°C and a humidity of 90%. The resulting cosmetic sheet was air-tightly stored in a storing bag formed by laminating aluminum with a polyethylene film in order to prevent evaporation of moisture in the sheet.

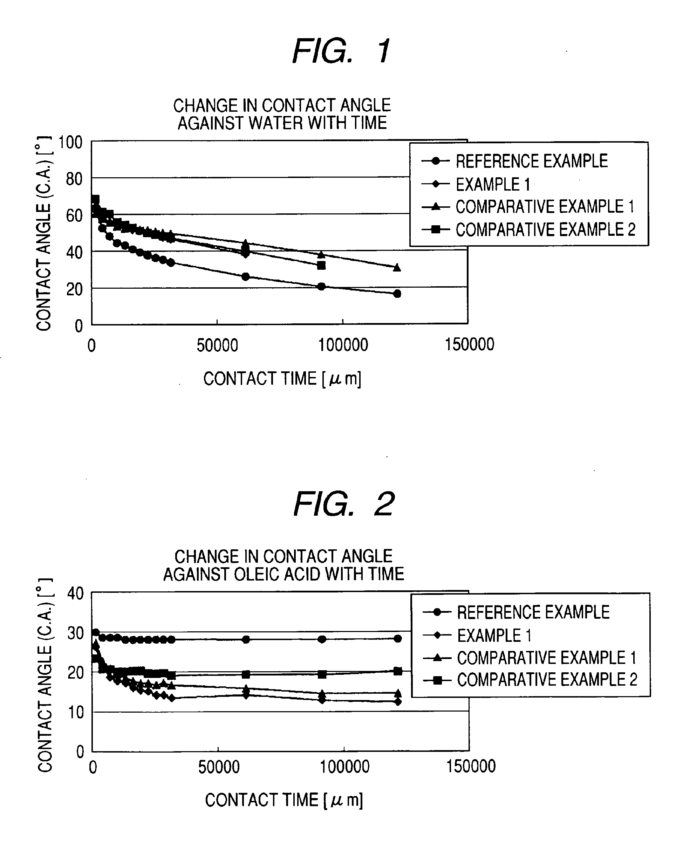

The presence of ooze-out of the polyoxyethylene sorbitan fatty acid ester on the surface of the skin-attaching layer of the resulting cosmetic sheet was judged by measuring a contact angle on the surface of the skin-attaching layer. Namely, in order to confirm the conditions of the surface of the skin-attaching layer, the measurement of the contact angle was carried out. By measuring a contact angle, information of a (solid) surface can be obtained. In this example, using a contact angle-measuring apparatus, measurement against water and measurement against oil (oleic acid) were conducted and change in contact angle with time was shown in graphs. Fig. 1 shows change in contact angle against water with time on the surface of the skin-attaching layer and Fig. 2 shows change In contact angle against an oil (oleic acid) with time on the surface of the skin-attaching layer. For reference, change in contact angle against water with time and change in contact angle against oleic acid with time on a commercially available cosmetic sheet for keratotic plug-removal (Reference Example) were measured and were shown in Fig. 1 and Fig. 2. In the case that the polyoxyethylene sorbitan fatty acid ester oozes out, it is considered that change in contact angle against water with time is large and change in contact angle against oleic acid with time increases.

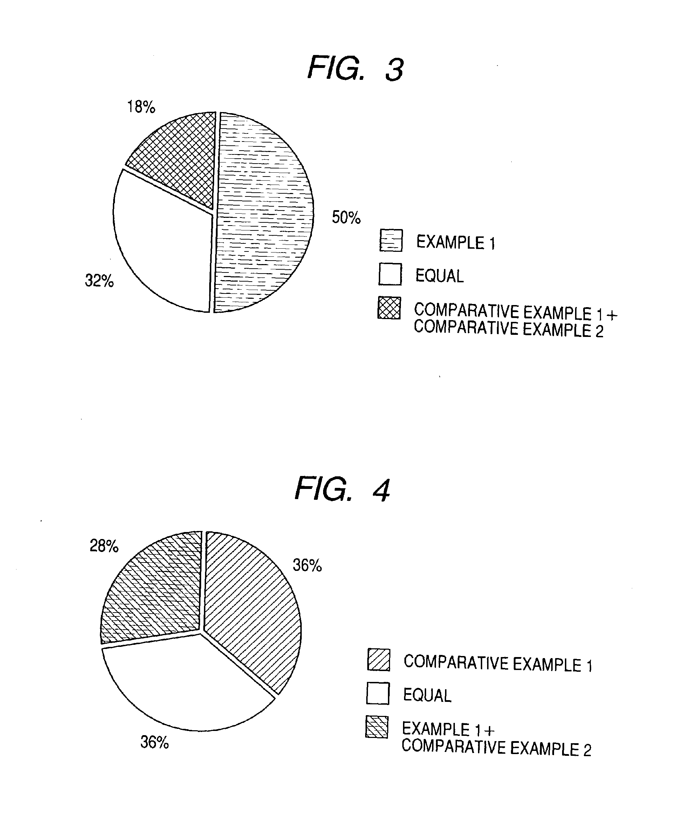

In addition, evaluation of the resulting cosmetic sheet on the keratotic plug-removing ability was conducted as shown below. The results are shown in Fig. 3 to Fig. 5.

(Comparative Example 1)

Using 57% by weight of polyvinylpyrrolidone having a weight-average molecular weight of 1,200,000 as a water-soluble polymer, 6% by weight of a sorbitan oleic acid monoester (ethylene oxide 20 mol adduct, HLB 15.0) as a polyoxyethylene sorbitan fatty acid ester, 34% by weight of silicic anhydride, and 3% by weight of triiron tetroxide, these compounds were stirred and mixed with an appropriate amount of purified water to prepare a coating solution. The coating solution was applied on the release-treated surface of a polyethylene film (thickness 50 $\mu$m) whose one surface had been subjected to silicone treatment so as to achieve an applied amount of 110 g/m$^2$ and a uniform thickness, whereby a skin-attaching layer was formed. A spun lace non-woven fabric (basis weight: 40 g/m$^2$) made of polyester fibers was overlaid on the skin-attaching layer and the whole was dried at 105°C for 3 minutes to prepare a cosmetic sheet having a three-layer structure. The moisture content of the resulting cosmetic sheet was found to be from 15 to 20% by weight. The moisture content was calculated based on the weight change after moisture control for about 15 minutes under the environment of a temperature of 30°C and a humidity of 90%. The resulting cosmetic sheet was air-tightly stored in a storing bag formed by laminating aluminum with a polyethylene film in order to prevent evaporation of moisture in the sheet.

In order to confirm the presence of ooze-out of the polyoxyethylene sorbitan fatty acid ester on the surface of the skin-attaching layer of the resulting cosmetic sheet, the measurement of a contact angle was carried out. Namely, using a contact angle-measuring apparatus, measurement against water and measurement against an oil (oleic acid) were conducted and change in contact angle with time was shown in graphs. Fig. 1 shows change in contact angle against water with time on the surface of the skin-attaching layer and Fig. 2 shows change in contact angle against an oil (oleic acid) with time on the surface of the skin-attaching layer.

In addition, evaluation of the resulting cosmetic sheet on the keratotic plug-removing ability was conducted as shown below. The results are shown in Fig. 3 to Fig. 5.

(Comparative Example 2)

Using 51% by weight of polyvinylpyrrolidone having a weight-average molecular weight of 1,200,000 as a water-soluble polymer, 15% by weight of a sorbitan oleic acid monoester (ethylene oxide 20 mol adduct, HLB 15.0) as a polyoxyethylene sorbitan fatty acid ester, 31% by weight of silicic anhydride, and 3% by weight of calcium carbonate, these compounds were stirred and mixed with an appropriate amount of purified water to prepare a coating solution. The coating solution was applied on the release-treated surface of a polyethylene film (thickness 50 $\mu$m), whose one surface had been subjected to silicone treatment, so as to achieve an applied amount of 110 g/m$^2$ and a uniform thickness, whereby a skin-attaching layer was formed. A spun lace non-woven fabric (basis weight: 40 g/m$^2$) made of polyester fibers was overlaid on the skin-attaching layer and the whole was dried at 105°C for 3 minutes to prepare a cosmetic sheet having a three-layer structure. The moisture content of the resulting cosmetic sheet was found to be from 15 to 20% by weight. The moisture content was calculated based on the weight change after moisture control for about 15 minutes under the environment of a temperature of 30°C and a humidity of 90%. The resulting cosmetic sheet was air-tightly stored in a storing bag formed by laminating aluminum with a polyethylene film in order to prevent evaporation of moisture in the sheet.

In order to confirm the presence of ooze-out of the polyoxyethylene sorbitan fatty acid ester on the surface of the skin-attaching layer of the resulting cosmetic sheet, the measurement of a contact angle was carried out. Namely, using a contact angle-measuring apparatus, measurement against water and measurement against an oil (oleic acid) were conducted and change in contact angle with time was shown in graphs. Fig. 1 shows change in contact angle against water with time on the surface of the skin-attaching layer and Fig. 2 shows change in contact angle against an oil (oleic acid) with time on the surface of the skin-attaching layer.

In addition, evaluation of the resulting cosmetic sheet on the keratotic plug-removing ability was conducted as shown below. The results are shown in Fig. 3 to Fig. 5.

As is apparent from Fig. 1, the contact angle on the cosmetic sheet of Example 1 decreases for a short period of time, so that it is found that wettability with water is satisfactory and affinity to water is excellent. Moreover, from Fig. 2, the contact angle against oleic acid on the cosmetic sheet of Example 1 decreases for a short period of time, so that it is found that wettability is better than that in any of Comparative Example 1, Comparative Example 2, and Reference Example and thus affinity to oil is high. Namely, from Fig. 1 and Fig. 2, it is found that the skin-attaching layer of the cosmetic sheet of Example 1 is excellent in the penetrating rate of water into the attaching layer, i.e., solubility In water and also affinity to oil is high. Since the polyoxyethylene sorbitan fatty acid ester is reserved on' the surface of the skin-attaching layer of the cosmetic sheet of Example 1, affinity to water and affinity to oil are both high. As a result, it is presumed that a skin surface showing oily nature is rapidly wetted and the keratotic plug-removing ability is enhanced. On the other hand, Reference Example is excellent In affinity to water but poor in affinity to oil, so that it is poor in affinity to a skin surface showing oily nature.

The evaluation method used in the above Examples is shown below.

[Evaluation Method for Keratotic Plug-Removing Ability (1)]

A cosmetic sheet was cut into an appropriate size (2.5 cm x 3.0 cm). The test piece of the cosmetic sheet was attached to one nose part of each of 60 healthy adult volunteers after an appropriate amount of water was applied thereto. It was allowed to stand in such an attached state for about 10 minutes and, after the cosmetic sheet was dried, it was peeled off. The number of keratotic plugs attached to the shin-attaching layer of the cosmetic sheet peeled was counted for each volunteer.

The test was carried out with dividing 60 volunteers into three groups: in the first group, the cosmetic sheet of Example 1 was attached on a half of one nostril and the cosmetic sheet of Comparative Example 1 on a half of the other nostril; in the second group, the cosmetic sheet of Comparative Example 1 was attached on a half of one nostril and the cosmetic sheet of Comparative Example 2 on a half of the other nostril; and in the third group, the cosmetic sheet of Comparative Example 2 was attached on a half of one nostril and the cosmetic sheet of Example 1 on a half of the other nostril.

Using the number of removed keratotic plugs determined by the above method, the following evaluation was conducted. Namely, using the number of removed keratotic plugs with the cosmetic sheet of Comparative Example 1 or 2 and the number of removed keratotic plugs with the cosmetic sheet of Example 1, evaluation was carried out based on the following judgment standard. The results are shown in Fig. 3. The resulting data of Comparative Examples 1 and 2 are shown in Fig. 3 in a combined form.

In the case that the number of keratotic plugs in Comparative Example 1 or 2/the number of keratotic plugs in Example 1 is 1.2 or more, Comparative Example 1 or 2 is superior.

In the case that the number of keratotic plugs in Comparative Example 1 or 2/the number of keratotic plugs in Example 1 is 0.8 or less, Example 1 is superior.

In the case that the number of keratotic plugs in Comparative Example 1 or 2/the number of keratotic plugs in Example 1 is larger than 0.8 and smaller than 1.2, the case is judged to be equal.

[Evaluation Method for Keratotic Plug-Removing Ability (2)]

The number of keratotic plugs in Example 1 or Comparative Example 2 was evaluated based on the number of keratotic plugs in Comparative Example 1 in the same manner as in the above evaluation method for keratotic plug-removing ability (1). The results are shown in Fig. 4. The resulting data of Example 1 and Comparative Example 2 are shown in Fig. 4 in a combined form.

[Evaluation Method for Keratotic Plug-Removing Ability (3)]

The number of keratotic plugs in Example 1 or Comparative Example 1 was evaluated based on the number of keratotic plugs in Comparative Example 2 in the same manner as in the above evaluation method for keratotic plug-removing ability (1). The results are shown in Fig. 5. The resulting data of Example 1 and Comparative Example 1 are shown in Fig. 5 in a combined form.

From Fig. 3 to FIG. 5, the following are found. Namely, the cases where the cosmetic sheet of Example 1 is superior account for 50% in Fig. 3, the cases where the cosmetic sheet of Comparative Example 1 is superior account for 36% in Fig. 4, and the cases where the cosmetic sheet of Comparative Example 2 is superior account for 13% in Fig. 5. Therefore, it was found that the cosmetic sheet of Example 1, i.e., the cosmetic sheet wherein the polyoxyethylene sorbitan fatty acid ester oozed out was judged superior to those of Comparative Examples 1 and 2.

Moreover, as is apparent from Comparative Example 2, it was found that, even when a large amount of the polyoxyethylene sorbitan fatty acid ester was added to the wet pressure-sensitive adhesive composition having a stable skin-attaching layer using a filler having a good affinity to the polyoxyethylene sorbitan fatty acid ester, the ester did not ooze out and hence it was impossible to enhance keratotic plug-removing ability, so that it was probable that the removing ability was rather judged to be decreased.

(Example 2)

Using 56% by weight of polyvinylpyrrolidone having a weight-average molecular weight of 1,200,000 as a water-soluble polymer, 6% by weight of a sorbitan oleic acid monoester (ethylene oxide 20 mol adduct, HLB 15.0) as a polyoxyethylene sorbitan fatty acid ester, 34% by weight of silicic anhydride, 1% by weight of titanium oxide, and 3% by weight of medicated carbon, these compounds were stirred and mixed with an appropriate amount of purified water to prepare a coating solution. The coating solution was applied on the release-treated surface of a polyethylene film (thickness 50 $\mu$m), whose one surface had been subjected to silicone treatment, so as to achieve an applied amount of 110 g/m$^2$ and a uniform thickness, whereby a skin-attaching layer was formed. A spun lace non-woven fabric (basis weight: 40 g/m$^2$) made of polyester fibers was overlaid on the skin-attaching layer and the whole was dried at 105°C for 3 minutes to prepare a cosmetic sheet having a three-layer structure. The moisture content of the resulting cosmetic sheet was found to be from 15 to 20% by weight. The moisture content was calculated based on the weight change after moisture control for about 15 minutes under the environment of a temperature of 30°C and a humidity of 90%. The resulting cosmetic sheet was air-tightly stored in a storing bag formed by laminating aluminum with a polyethylene film in order to prevent evaporation of moisture in the sheet.

Using the cosmetic sheet of the above Example 2 and Comparative Example 1, confirmation of the presence of uneven distribution in the skin-attaching layer and evaluation on the keratotic plug-removing ability were conducted as shown below.

[Test for Confirming Presence of Uneven Distribution in Skin-Attaching Layer]

As a test sample, the cosmetic sheet was cut into an appropriate size (1.0 cm x 7.0 cm) to prepare test pieces. The resulting test piece was attached to a dedicated cell made of zinc selenide for a Fourier transform infrared spectrophotometer manufactured by Simadzu Corporation and was peeled off after one minute of standing. The dedicated cell was measured within the wavelength range of 400 to 400 cm$^{-1}$, whereby the presence of the peaks of the sorbitan oleic acid monoester was confirmed.

For the cosmetic sheet of Example 2, it was possible to confirm the peaks of the sorbitan oleic acid monoester. On the other hand, for the cosmetic sheet of Comparative Example 1, it was impossible to confirm the peaks of the sorbitan oleic acid monoester. Namely, it was found that the sorbitan oleic acid monoester was unevenly distributed at the surface part of the skin-attaching layer of the cosmetic sheet of Example 2 but the sorbitan oleic acid monoester was not unevenly distributed at the surface part of the skin-attaching layer of the cosmetic sheet of Comparative Example 1.

[Evaluation Method for Keratotic Plug-Removing Ability (4)]

As a test sample, the cosmetic sheet was cut into an appropriate size (2.5 cm x 3.0 cm) to prepare test pieces. The test piece of the cosmetic sheet was attached to one nose part of each of 60 healthy adult volunteers after an appropriate amount of water was applied thereto. It was allowed to stand in such an attached state for about 10 minutes and, after the cosmetic sheet was dried, it was peeled off. The number of keratotic plugs attached to the skin-attaching layer of the cosmetic sheet peeled was counted for each volunteer.

The test was carried out by attaching the cosmetic sheet of Example 2 on a half of one nostril and the cosmetic sheet of Comparative Example 1 on a half of the other nostril.

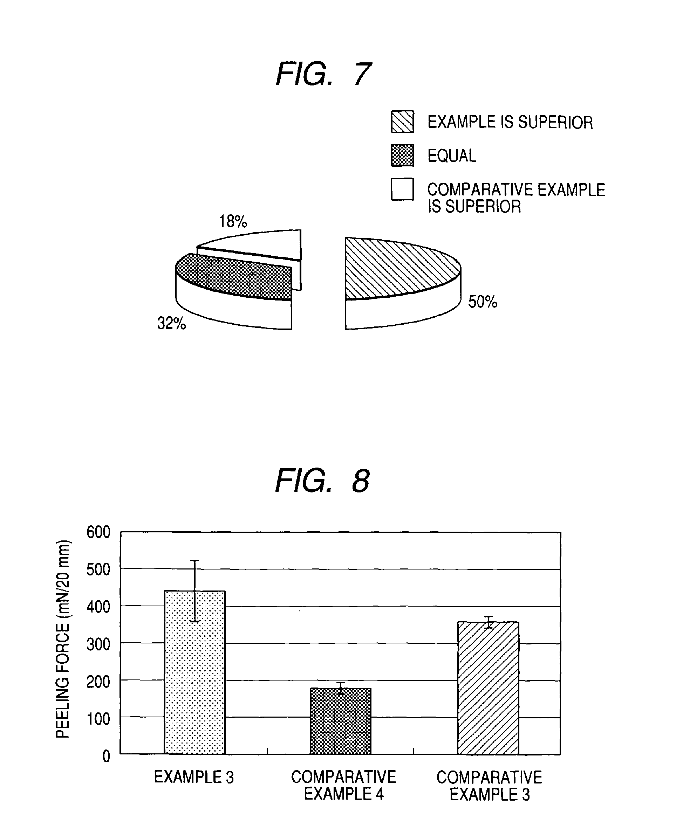

For each volunteer, using the number of removed keratotic plugs with the cosmetic sheet of Example 2 and the number of removed keratotic plugs with the cosmetic sheet of Comparative Example 1, evaluation was carried out based on the following judgment standard. The results are shown in Fig. 7.

In the case that the number of keratotic plugs in Example 2/the number of keratotic plugs in Comparative Example 1 is 1.2 or more, Example 2 is superior.

In the case that the number of keratotic plugs in Example 2/the number of keratotic plugs in Comparative Example 1 is 0.8 or less, Comparative Example 1 is superior.

In the case that the number of keratotic plugs in Example 2/the number of keratotic plugs in Comparative Example 1 is larger than 0.8 and smaller than 1.2, the case is judged to be equal.

As is apparent from Fig. 7, the cases where the cosmetic sheet of Example 2 is superior account for 50%, the cases where the cosmetic sheet of Comparative Example 1 is superior account for 18%, and the cases where they are equal account for 32%. Therefore, it was found that the cosmetic sheet of Example 2, i.e., the cosmetic sheet wherein the sorbitan oleic acid monoester was unevenly distributed at the surface part of the skin-attaching layer was judged superior in keratotic plug-removing ability to the cosmetic sheet of Comparative Example 1 wherein the monoester was not unevenly distributed at the surface part.

Moreover, the cosmetic sheet of Example 2 which contains the polyoxyethylene sorbitan fatty acid ester in the skin-attaching layer could reduce pain at the time of peeling from the skin.

(Example 3)

Using 58% by weight of polyvinylpyrrolidone having a weight-average molecular weight of 1,200,000 as a water-soluble polymer, 6% by weight of a sorbitan oleic acid monoester (ethylene oxide 20 mol adduct, HLB 15.0) as a polyoxyethylene sorbitan fatty acid ester, 34% by weight of silicic anhydride, and 2% by weight of titanium oxide, these compounds were stirred and mixed with an appropriate amount of purified water to prepare a coating solution. The coating solution was applied on the release-treated surface of a polyethylene film (thickness 50 $\mu$m) whose one surface had been subjected to silicone treatment so as to achieve an applied amount of 110 g/m$^2$ and a uniform thickness, whereby a skin-attaching layer was formed. A spun lace non-woven fabric (basis weight: 40 g/m$^2$) made of polyester fibers was overlaid on the skin-attaching layer and the whole was dried at 100°C for 1.5 minutes to prepare a cosmetic sheet having a three-layer structure. The moisture content of the resulting cosmetic sheet was found to be from 15 to 20% by weight. The moisture content was calculated based on the weight change after stored at room temperature for about 24 hours. The resulting cosmetic sheet was air-tightly stored in a storing bag formed by laminating aluminum with a polyethylene film in order to prevent evaporation of moisture in the sheet.

(Comparative Example 3)

A coating solution was prepared in the same manner as in Example 3 except that the polyoxyethylene sorbitan fatty acid ester was not used and 6% by weight of glycerol was used instead. A cosmetic sheet was prepared in the same manner as in Example 3 except that the coating solution was applied on the silicone-treated surface of a polyethylene film (thickness 50 $\mu$m) whose one surface had been subjected to silicone treatment. The moisture content of the resulting cosmetic sheet was found to be from 15 to 20% by weight. The resulting cosmetic sheet was stored in the same manner as in Example 3.

Using a commercially available cosmetic sheet as Reference Example, keratotic plug-removing ratio of the cosmetic sheets prepared in Example 3 and Comparative Example 3 was determined as shown below.

Namely, each of the cosmetic sheets of Example 3, Comparative Example 3, and Reference Example was cut into an appropriate size (2.5 cm x 3.0 cm). The test piece of the cosmetic sheet was attached to one nose part of each of 60 healthy adult volunteers after an appropriate amount of water was applied thereto. It was allowed to stand in such an attached state for about 10 minutes and, after the cosmetic sheet was dried, it was peeled off. The number of keratotic plugs attached to the akin-attaching layer of the cosmetic sheet peeled was counted for each volunteer.

Then, the keratotic plug-removing ratio in Example 3 or Comparative Example 3 was determined, while the number of keratotic plugs removed using the cosmetic sheet of Reference Example was regarded as 1. Namely, the keratotic plug-removing ratio was determined by dividing the number of keratotic plugs removed using the cosmetic sheet of Example 3 or Comparative Example 3 by the number of keratotic plugs removed using the cosmetic sheet of Reference Example. The keratotic plug-removing ratio in each of Example 3 and Comparative Example 3 were shown as an average value of values obtained.

**Keratotic plug-removing ratio = Number of keratotic plugs removed in Example 3 or Comparative Example 3/Number of keratotic plugs removed in Reference Example**

The test was carried out with dividing 60 volunteers into three groups: in the first group, the cosmetic sheet of Example was attached on a half of one nostril and the cosmetic sheet of Reference Example on a half of the other nostril; in the second group, the cosmetic sheet of Comparative Example was attached on a half of one nostril and the cosmetic sheet of Example on a half of the other nostril; and in the third group, the cosmetic sheet of Reference Example was attached on a half of one nostril and the cosmetic sheet of Comparative Example on a half of the other nostril.

As results of above evaluation, there were obtained the results that the keratotic plug-removing ratio in Example 3 was found to be 1.3 and the keratotic plug-removing ratio in Comparative Example 3 was found to be 0.95. Namely, Example 3, the sheet of the present invention, showed a keratotic plug-removing ratio 1.3 times larger than that in Reference Example but the keratotic plug-removing ratio was 0.95 in Comparative Example 3, which only showed a keratotic plug-removing ability almost equal to that in Reference Example.

(Comparative Example 4)

A coating solution was prepared in the same manner as in Example 3. A cosmetic sheet was prepared in the same manner as in Example 3 except that the coating solution was applied on one surface of a polyethylene film (thickness 50 $\mu$m), whose one surface had been subjected to silicone treatment, so as to achieve an applied amount of 110 g/m$^2$ and a uniform thickness to form a skin-attaching layer. The moisture content of the resulting cosmetic sheet was found to be from 15 to 20% by weight. The resulting cosmetic sheet was stored in the same manner as in Example 3.

Using the cosmetic sheets of Example 3, Comparative Example 3, and Comparative Example 4, separator peeling force was measured by the method shown below. The results are shown in the graph of Fig. 8.

[Method for Measuring Separator Peeling Force]

As a test sample, the cosmetic sheet was cut into three sheets of test pieces having a width of 20 mm and a length of 10 mm. The backside of the test piece at the separator side was adhered under pressure to a two-sided tape part of a phenol resin laminate plate to which a two-sided tape had been attached, by means of a roller (pressure: 2 kg). Then, using a tensile testing machine, an edge part of the test piece was drawn under conditions of a drawing angle of 90° and a drawing rate of 300 mm/min to measure peeling force of the test piece. The peeling force was determined as an average value on three sheets of the test pieces.

As is apparent form Fig. 8, the peeling force in Example 3 was found to be 438 mN/20 mm and the peeling force in Comparative Example 4 was found to be 181 mN/20 mm. Namely, it was found that the cosmetic sheet of Example 3 had a peeling force 2.4 times larger than that of Comparative Example 4. Moreover, since a layer of the polyoxyethylene sorbitan fatty acid ester was not formed on the surface of the skin-attaching layer in the cosmetic sheet of Comparative Example 3, the separator peeling force was found to be 356 mN/20 mm. It was found that the inventive cosmetic sheet of Example 3 had a separator peeling force almost equal to or slightly larger than that of the cosmetic sheet of Comparative Example 3 and resulted in no light peeling.

Namely, according to the present invention, a cosmetic sheet can be obtained, wherein a polyoxyethylene sorbitan fatty acid ester is present on the surface of the skin-attaching layer and enhancement of keratotic plug-removing ability can be realized and which can be cut into any shape without resulting light peeling at its production, also results no light peeling even during storage, and can protect the skin-attaching layer until its use from the viewpoint of hygiene and adhesive force. Moreover, incorporation of the polyoxyethylene sorbitan fatty acid ester in the skin-attaching layer could reduce the pain at the time of peeling off from the skin.

The cosmetic sheet of the present invention is useful as a keratotic plug-removing sheet, an acne sheet, or the like. In particular, it can be effectively utilized as a keratotic plug-removing sheet at the nose part and the like.

While the invention has been described in detail and with reference to specific examples thereof, it will be apparent to one skilled in the art that various changes and modifications can be made therein without departing from the scope thereof.

The present application is based on Japanese Patent Applications No. 2005-135722 filed May 9, 2005, No. 2005-235286 filed August 15, 2005, and No. 2005-255270 filed September 2, 2005, the entire contents thereof being hereby incorporated by reference.

**Claims**

1. A cosmetic sheet which comprises a support and, provided on one surface of the support, a skin-attaching layer comprising a wet pressure-sensitive adhesive composition containing a water-soluble polymer and a polyoxyethylene sorbitan fatty acid ester, wherein the polyoxyethylene sorbitan fatty acid ester has oozed out on the surface of the skin-attaching layer.

2. The cosmetic sheet according to claim 1, wherein the polyoxyethylene sorbitan fatty acid ester which has oozed out on the surface of the skin-attaching layer exists as a layer.

3. The cosmetic sheet according to claim 1, wherein said wet pressure-sensitive adhesive composition further comprises a filler.

4. The cosmetic sheet according to claim 3, wherein said filler is at least one selected from the group consisting of silica, alumina, titanium dioxide, titanium black, carbon black, black iron oxide, medicated carbon, aluminum hydroxide, magnesium hydroxide, calcium carbonate, and talc.

5. The cosmetic sheet according to claim 3, wherein said inorganic filler has an average particle size of 0.01 to 50 $\mu$m.

6. The cosmetic sheet according to claim 1 or 2, wherein the fatty acid forming said polyoxyethylene sorbitan fatty acid ester is one selected from the group consisting of lauric acid, palmitic acid, stearic acid, and oleic acid.

7. The cosmetic sheet according to claim 1 or 2, wherein HLB of said polyoxyethylene sorbitan fatty acid ester is 12 or more.

8. The cosmetic sheet according to claim 1, wherein said water-soluble polymer is one selected from the group consisting of polyvinylpyrrolidone and vinylpyrrolidone-vinyl acetate copolymers.

9. The cosmetic sheet according to claim 1, wherein said water-soluble polymer comprises a water-soluble polymer having a weight-average molecular weight of less than 700,000 and a water-soluble polymer having a weight-average molecular weight of 700,000 or more.

10. The cosmetic sheet according to claim 1, wherein said wet pressure-sensitive adhesive composition further comprises a moisturizing component.

11. The cosmetic sheet according to claim 10, wherein said moisturizing component is one selected from the group consisting of glycerol and polyhydric alcohols.

12. The cosmetic sheet according to claim 1, wherein said support is a moisture-permeable support.

13. The cosmetic sheet according to claim 1, which further comprises a sheet as a separator on the surface of said skin-attaching layer.

14. The cosmetic sheet according to claim 13, wherein the sheet as a separator is a sheet which has not been subjected to release treatment.

15. The cosmetic sheet according to claim 13, wherein said sheet is one selected from the group consisting of plastic films and paper.

**16.** The cosmetic sheet according to claim 14, wherein said plastic film comprises one selected from the group consisting of polyethylene, polypropylene, and polyesters.

## FIG. 1

CHANGE IN CONTACT ANGLE
AGAINST WATER WITH TIME

## FIG. 2

CHANGE IN CONTACT ANGLE
AGAINST OLEIC ACID WITH TIME

## FIG. 3

18%

50%

32%

☰ EXAMPLE 1

☐ EQUAL

▧ COMPARATIVE EXAMPLE 1 +
COMPARATIVE EXAMPLE 2

## FIG. 4

28%

36%

36%

▨ COMPARATIVE EXAMPLE 1

☐ EQUAL

▨ EXAMPLE 1 +
COMPARATIVE EXAMPLE 2

## FIG. 5

13%

51%

36%

▨ COMPARATIVE EXAMPLE 2

☐ EQUAL

▧ COMPARATIVE EXAMPLE 1 +
EXAMPLE 1

## FIG. 6

1

2

3

SURFACE TO BE
ATTACHED TO SKIN

# FIG. 7

EXAMPLE IS SUPERIOR

EQUAL

COMPARATIVE EXAMPLE IS SUPERIOR

18%

32%

50%

# FIG. 8

**European Patent Office**

**EUROPEAN SEARCH REPORT**

Application Number

EP 06 00 8975

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| P,X | PATENT ABSTRACTS OF JAPAN vol. 2003, no. 12, 5 December 2003 (2003-12-05) -& JP 2005 314237 A (NITTO DENKO CORP), 10 November 2005 (2005-11-10) * abstract * | 1-16 | INV. A61Q19/00 A61K8/02 A61K8/37 A61K8/81 |
| X | US 6 159 493 A (CHEN ET AL) 12 December 2000 (2000-12-12) * the whole document * | 1,6,8, 10-12 | |
| A | EP 0 947 189 A (NITTO DENKO CORPORATION (JP)) 6 October 1999 (1999-10-06) * the whole document * | 1-16 | |
| A | EP 0 965 332 A (LINTEC CORPORATION (JP)) 22 December 1999 (1999-12-22) * the whole document * | 1-16 | |
| A | US 6 174 536 B1 (CROTTY BRIAN ANDREW ET AL) 16 January 2001 (2001-01-16) * the whole document * | 1-16 | TECHNICAL FIELDS SEARCHED (IPC) A61K |
| A | US 5 985 299 A (BUERGER ET AL) 16 November 1999 (1999-11-16) * the whole document * | 1-16 | |
| A | US 6 306 382 B1 (UEMURA TOMOHIRO ET AL) 23 October 2001 (2001-10-23) * the whole document * | 1-16 | |

The present search report has been drawn up for all claims

| Place of search | Date of completion of the search | Examiner |
|---|---|---|
| Munich | 10 August 2006 | Diebold, A |

EPO FORM 1503 03.82 (P04C01)

## ANNEX TO THE EUROPEAN SEARCH REPORT
## ON EUROPEAN PATENT APPLICATION NO.

EP 06 00 8975

This annex lists the patent family members relating to the patent documents cited in the above-mentioned European search report.
The members are as contained in the European Patent Office EDP file on
The European Patent Office is in no way liable for these particulars which are merely given for the purpose of information.

10-08-2006

| Patent document cited in search report | | Publication date | Patent family member(s) | | Publication date |
|---|---|---|---|---|---|
| JP 2005314237 | A | 10-11-2005 | NONE | | |
| US 6159493 | A | 12-12-2000 | TW | 393321 B | 11-06-2000 |
| EP 0947189 | A | 06-10-1999 | AT | 292949 T | 15-04-2005 |
| | | | DE | 69733028 D1 | 19-05-2005 |
| | | | WO | 9806375 A1 | 19-02-1998 |
| | | | KR | 2000029965 A | 25-05-2000 |
| | | | US | 2003044599 A1 | 06-03-2003 |
| EP 0965332 | A | 22-12-1999 | AU | 3317599 A | 23-12-1999 |
| | | | CA | 2274294 A1 | 10-12-1999 |
| | | | CN | 1243705 A | 09-02-2000 |
| | | | DE | 69912683 D1 | 18-12-2003 |
| | | | DE | 69912683 T2 | 16-09-2004 |
| | | | KR | 2000006074 A | 25-01-2000 |
| | | | US | 2002022011 A1 | 21-02-2002 |
| US 6174536 | B1 | 16-01-2001 | NONE | | |
| US 5985299 | A | 16-11-1999 | NONE | | |
| US 6306382 | B1 | 23-10-2001 | NONE | | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 5221843 A
- JP 11199435 A
- JP 2005135722 A
- JP 2005235286 A
- JP 2005255270 A